# EUROPEAN PATENT APPLICATION

(11) **EP 4 299 056 A1**
(43) Date of publication of application: **03.01.2024**
(21) Application number: 22857815.9
(22) Date of filing: 16.08.2022
(51) Int. Cl.: A61K 9/22, A61K 31/343, A61P 25/20

(54) **SLEEP REGULATION TABLET ALLOWING RELEASE BY STAGE AND PREPARATION METHOD THEREFOR**

(30) Priority: 18.08.2021 CN 202110950477
(71) Applicant: Overseas Pharmaceuticals, Ltd., China 510530 (CN)
(72) Inventor: WEN, Xiaoguang, Guangzhou, Guangdong 510000 (CN); WANG, Jingya, Guangzhou, Guangdong 510000 (CN); ZHAO, Dachuan, Guangzhou, Guangdong 510000 (CN); FAN, Jun, Guangzhou, Guangdong 510000 (CN); ZHANG, Chenliang, Guangzhou, Guangdong 510000 (CN); WANG, Peipei, Guangzhou, Guangdong 510000 (CN)
(74) Representative: Murgitroyd & Company
(86) International application number: PCT/CN2022/112853
(87) International publication number: WO 2023/020504

(57) **Abstract**

The present disclosure provides "a sleep-regulating tablet allowing release by stages and a preparation method thereof", belongs to a pharmaceutical technology, and is characterized in that each tablet structurally consists of a drug-containing delayed-release tablet core, a drug-free stomach-soluble coating, a drug-free enteric coating, a drug-containing immediate-release shell, and a shell stomach-soluble coating sequentially from inside to outside. An ideal dual-stage timed drug release mode may be realized, and is especially suitable for a sleep-regulating drug, such as ramelteon.

## Description

### Technical Field

The present disclosure relates to a pharmaceutical technology and particularly to a sleep-regulating tablet allowing release by stages and a preparation method therefor.

### Background

Some drugs such as sleep-regulating drugs need to be rapidly released and take effect quickly after being taken by a patient, but a blood concentration rapidly fluctuates and an adverse reaction rate is high. A preparation technology needs to be developed to ensure that the blood concentration of the drugs *in vivo* is more stable, a pharmaceutical effect disappears later, and the adverse reaction rate is lower.

Ramelteon (Rozerem^{®}) is an oral hypnotic drug developed by Japan Takeda company and approved to be on the market by the U.S. FDA on July 22, 2005. The ramelteon is a first melatonin receptor agonist applied to a clinical treatment of insomnia, and mainly used for treating difficult-sleeping type insomnia, and has an exact curative effect on chronic insomnia and short-term insomnia. The ramelteon is a first insomnia therapeutic prescription drug with a new therapeutic action mechanism in the last 35 years.

The action mechanism of the current hypnotics is generally to inhibit a brain nerve activity of a human body in a large range. The ramelteon has a unique pharmacological effect, namely selectively acting on two receptors in human hypothalamic suprachiasmatic nucleus (SCN). The SCN is known as a "biological clock" which regulates a periodic biological rhythm of human body over 24 hours, including a sleep-wake period. A study result shows that adverse reactions such as overuse, a drug withdrawal reaction, drug dependence, "hangover" (after-effect on the next day) and the like may not occur after the drug is used. A result of a phase III clinical study shows that the ramelteon may effectively shorten the time for a patient to fall asleep, increase the total sleep time, and improve the sleep efficiency, has a small negative influence on work and study in the next day, and also has a very low recurrence rate after drug withdrawal. In 1 study, 472 patients took the product 1 time a day for up to 1 year. According to results of recent clinical studies, the product is safe to take by elderly patients, patients with mild-moderate obstructive pulmonary disease (COPD), and patients with mild-moderate sleep apnea.

Most of the ramelteon preparations in the current market are immediate-release preparations which are released only once, may take effect quickly, and help to fall asleep quickly. But the ramelteon preparations may not prevent an early awakening, have a high blood concentration Cₘₐₓ *in vivo,* and are easy to improve an incidence rate of adverse reactions.

Although there are references describe a sleep-regulating drug with a dual-stage release mode, the preparation of the sleep-regulating drug is characterized in that a drug is prepared into an immediate-release pellet and a delayed-release pellet, the two pellets are mixed into a capsule or are compressed into a tablet together with a filler, and the two pellets realize a dual-stage release based on different dissolution characteristics, for example, "timed dual-release dosage form containing short-acting hypnotic or salt thereof' as described in CN00809487. The prepared pellets and the filler are pressed into a tablet. Since a particle size difference is likely to cause an uneven mixing, a layering phenomenon may occur during the pressing process. Besides, during the pressing process, the pellets may be crushed to affect a final release. It is difficult to achieve an ideal two-stage release mode. Therefore, it is necessary to develop a new tablet and a tableting technology thereof.

### Summary

Based on the problems to be solved in the field, the present disclosure provides a tablet allowing release by two stages of a drug and a preparation method therefor. The tablet realizes an ideal dual-stage timed drug release mode, and is particularly suitable for a sleep-regulating drug such as ramelteon.

The technical solution of the present disclosure is as follows:
A tablet allowing sleep-regulating active ingredients release by stages , wherein each tablet structurally from inside to outside contains a drug-containing delayed-release tablet core, a drug-free enteric coating, a drug-containing immediate-release shell, and a shell stomach-soluble coating sequentially from inside to outside.

Preferably, the the drug-free enteric coating provides a weight gain by > 4%, (preferably by 4%-12%, or 5%, 6%, 7%, and 8%, on the basis of that of the drug-containing delayed-release tablet core and the drug-free enteric coating is soluble at a pH of 5 or more, preferably at a pH of 7 or more, such that 0.5-4 hours after the drug-containing immediate-release shell completely release, the drug-containing delayed-release tablet core can start to release and release completely within 0.1-6 hours , preferably in 0.5-4 hours.

Preferably, in the tablet, a drug-free stomach-soluble coating is arranged between the drug-containing delayed-release tablet core and the drug-free enteric coating, and the drug-free stomach-soluble coating provides weight gain by 2-5% on the basis of the drug-containing delayed-release tablet core, preferably provides weight gain by 3% or 4%.

Preferably, in the tablet, a film-forming material of the drug-free enteric coating is selected from acrylic resin I, II, and III, cellulose acetate phthalate (CAP), hydroxypropyl methylcellulose phthalate (HPMCP), polyvinyl acetate phthalate (PVAP), cellulose acetate trimellitate (CAT), acrylic resin EuS100 and EuL100, a methacrylic acid-methyl methacrylate copolymer, and a methacrylic acid-ethyl acrylate copolymer, and preferably, the used film-forming material is the methacrylic acid-ethyl acrylate copolymer.

Preferably, in the tablet, a content ratio of a pharmaceutically active ingredients in the drug-containing immediate-release shell to the drug-containing delayed-release tablet core is 1:1~3.

Preferably, in the tablet, the drug-containing delayed-release tablet core is an immediate-release preparation, in which, auxiliary materials and corresponding weight percentage as follows:
60-90% of a filler;
2-10%, preferably 4-8% of a disintegrant;
2-6%, preferably 2.5-5% of a binder;
0.01-1%, preferably 0.05-1% of an antioxidant; and
0.5-3%, preferably 0.5-1.5% of a lubricant or a flow aid,
wherein the filler and the antioxidant are internal auxiliary materials, the lubricant/the flow aid is an external auxiliary material, and the disintegrant can be as an internal auxiliary material or an external auxiliary material.

Preferably, in the tablet, the drug-containing delayed-release tablet core is a slow-release preparation, in which auxiliary materials and corresponding weight percentage arerespectively as follows:
60-90% of a filler;
2-6%, preferably 2.5-5% of a binder;
4-25%, preferably 5-20% of a slow-release material;
0.01-1%, preferably 0.05-1% of an antioxidant; and
0.5-3%, preferably 0.5-1.5% of a lubricant/a flow aid,
wherein the filler, the binder, and the antioxidant are internal auxiliary materials, and the lubricant/the flow aid is an external auxiliary material.

Preferably, in the tablet, a weight ratio of the drug-containing immediate-release shell to the drug-containing delayed-release tablet core is 4 or more , preferably in the range of 4-12, or is 350:60.

Preferably, in the tablet, the weight of the shell stomach-soluble coating provides weight gain by 2-5%,preferably provides weight gain by 3%.

The filler is one or more selected from microcrystalline cellulose, lactose, pregelatinized starch, starch, calcium hydrogen phosphate, mannitol, dextrin, etc.

The binder is selected from one or more of polyvidone, copovidone, hydroxypropyl cellulose, hydroxypropyl methylcellulose;
The disintegrant is selected from one or more of starch, pregelatinized starch, low-substituted hydroxypropyl cellulose, sodium carboxymethyl starch, crospovidone, croscarmellose sodium, etc.

The slow-release material is selected from one or more of hydroxypropyl cellulose, hydroxypropyl methylcellulose, alginate, ethyl cellulose, vinyl acetate, xanthan gum, guar gum, crospovidone, and glyceryl behenate.

The antioxidant is one or more selected from vitamin E, p-hydroxyanisole, butylated hydroxyanisole, dibutyl cresol, sodium bisulfite, sodium metabisulfite, and vitamin C.

The lubricant/the flow aid is selected from one or more of magnesium stearate, sodium stearyl fumarate, colloidal silicon dioxide, sodium lauryl sulfate, talcum powder, etc.

Preferably, the tablet is prepared by using the following steps:
(1) preparing a material of the drug-containing immediate-release shell: preparing a total mixed particle of the drug-containing immediate-release shell according to a formula;
(2) preparing the drug-containing delayed-release tablet core: preparing a total mixed particle of the drug-containing delayed-release tablet core, and preparing a tablet core by using a pressing method; and sequentially performing stomach-soluble coating and enteric coating on the tablet core to obtain the drug-containing delayed-release tablet core; and
(3) filling a part of the material of the drug-containing immediate-release shell into a middle die as a first layer, and placing the drug-containing delayed-release tablet core on the first layer to serve as a second layer, and then performing pre-pressing; and filling the rest material of the drug-containing immediate-release shell as a third layer, and performing main-pressing to obtain the tablet.

Preferably, in the tablet, the pre-pressing is performed at a pressure of 0.02-1.5 kN, and the main-pressing is performed at a main pressure of 15-35 kN.

Preferably, in any one of the tablet, the sleep-regulating active ingredients is ramelteon.

A method for preparing any one of the tablet, comprising the following steps:
(1) preparing a material of the drug-containing immediate-release shell: preparing a total mixed particle of the drug-containing immediate-release shell according to a formula;
(2) preparing the drug-containing delayed-release tablet core: preparing a total mixed particle of the drug-containing delayed-release tablet core, and preparing a tablet core by using a pressing method; and sequentially performing stomach-soluble coating and enteric coating on the tablet core to obtain the drug-containing delayed-release tablet core; and
(3) filling a part of the material of the drug-containing immediate-release shell into a middle die as a first layer; placing the drug-containing delayed-release tablet core on the first layer to serve as a second layer, and then performing pre-pressing; and filling the rest material of the drug-containing immediate-release shell as a third layer, and performing main-pressing to obtain the tablet.

Preferably, in the preparation method, the pre-pressing is performed at a pressure of 0.02-1.5 kN, and the main-pressing is performed at a main pressure of 15-35 kN.

The total mixed particle of the drug-containing delayed-release tablet core and the total mixed particle of the drug-containing immediate-release shell are prepared by using any one of the following methods:
method I: respectively weighing the pharmaceutically active ingredient and the various sieved internal auxiliary materials according to the prescription dosage, adding the pharmaceutically active ingredient and the antioxidant into a certain amount of a solvent to prepare a solution or a suspension, spraying the solution or the suspension into the rest internal auxiliary materials to complete a wet granulation, adding the external auxiliary materials after drying, and uniformly mixing the materials to obtain a corresponding total mixed particle;
method II: respectively weighing the pharmaceutically active ingredient and the various sieved internal auxiliary materials according to the prescription dosage, adding the pharmaceutically active ingredient into a certain amount of a solvent to prepare a solution or a suspension, spraying the solution or the suspension into the internal auxiliary materials to complete a wet granulation, adding the external auxiliary materials after drying, and uniformly mixing the materials to obtain a corresponding total mixed particle;
method III: respectively weighing the pharmaceutically active ingredient and the various sieved internal auxiliary materials according to the prescription dosage, adding the antioxidant and or the binder into a certain amount of a solvent to prepare a solution or a suspension, spraying the solution or the suspension into a mixed material of the pharmaceutically active ingredient and the rest internal auxiliary materials to complete a wet granulation, adding the external auxiliary materials after drying, and uniformly mixing the materials to obtain a corresponding total mixed particle; and
method IV: respectively weighing the pharmaceutically active ingredient and the various sieved internal auxiliary materials according to the prescription dosage, and uniformly mixing the pharmaceutically active ingredient and the auxiliary materials according to an equal amount adding method to obtain a corresponding total mixed particle.

Through studies on a medicinal tablet structure, a formula, and a tableting process, the present disclosure obtains a formula and a preparation technology of a tablet allowing release by stages of a medicinally active substance, particularly suitable for a sleep-regulating drug. The present disclosure makes up for the deficiency of the tablets in the prior art.

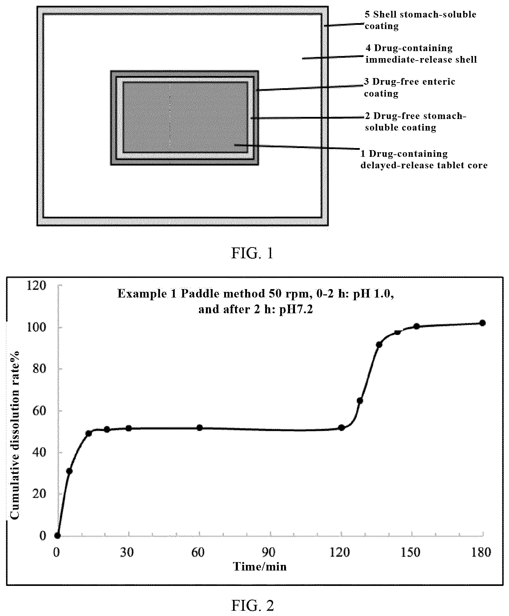

In a dissolution test of the tablet provided by the present disclosure, results were as shown in FIGs. 2, 4, and 5. The release of the drug-containing immediate-release shell is completed within 10-30 minutes. Due to the protection of the drug-free enteric coating, no drug is released within a period of time, such as 1.5-4 hours. Then a second stage is started, and the drug in the drug-containing delayed-release tablet core is released to realize a relatively ideal two-stage release mode. The tablet is used in a sleep-regulating drug and may quickly take effect after being taken to help fall asleep quickly. The second-stage release is started after a period of time, such that early awakening is prevented and the total sleep time is increased.

In order to obtain the tablet realizing the release mode, the present disclosure performs drug-free stomach-soluble coating and drug-free enteric coating on the drug-containing delayed-release tablet core in the tablet structure. In a research and development test of the present disclosure, since the enteric coating technology requires a relatively long time, the tablet core is likely to be broken easily in the enteric coating process. Therefore, preferably, the stomach-soluble coating may be performed to protect the tablet core before the enteric coating, and does not delay the release of the tablet core in an enteric environment.

In addition, the present disclosure studies an effect of a weight gaining thickness of the enteric coating on the release mode. It is found that if the enteric coating film is too thin, the tablet core cannot be prevented from releasing in a gastric acid environment. If the coating film is too thick, a process cost will be increased, and the release of the tablet core is delayed. It is found that an ideal weight-gaining range of the enteric coating is 4-12%.

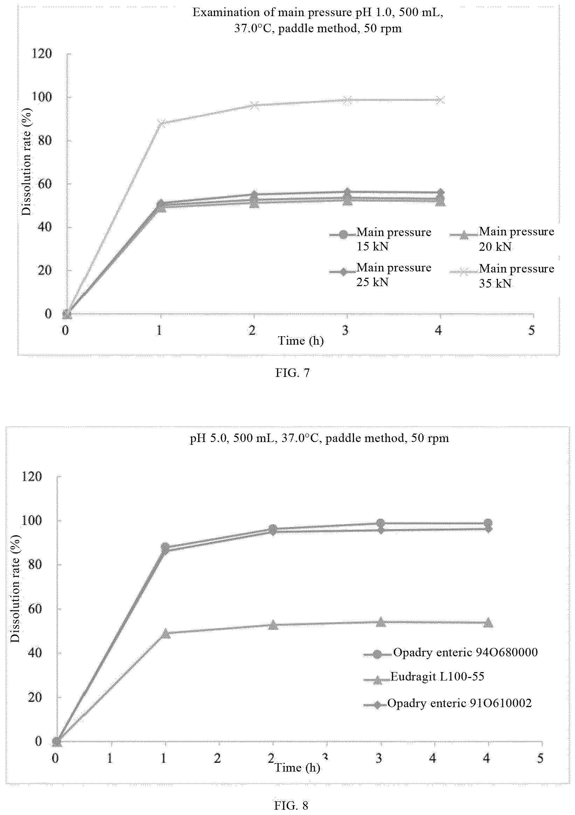

The study of the present disclosure also finds that since some enteric coating materials are not tough enough, the enteric coating is easy to crack or generate gaps in a tableting process so as to cause an early release of the delayed-release tablet core, or a part of the enteric coating materials are not suitable for a film-forming material and it may only ensure that the enteric coating materials are not dissolved below a pH of 4.0. For example, as shown in FIG. 7, when the coating material is Opadry enteric 94O680000 and 910610002, the tablet core is released in an environment of a pH of 5.0, and a dual-stage release may not be realized.

In order to obtain the tablet that achieves a targeted release mode of the present disclosure, the present study finds that when the film-forming material of the selected enteric coating material is one or more materials from the following group: acrylic resin I, II, and III, cellulose acetate phthalate (CAP), hydroxypropyl methylcellulose phthalate (HPMCP), polyvinyl acetate phthalate (PVAP), cellulose acetate trimellitate (CAT), acrylic resin EuS100 and EuL100, a methacrylic acid-methyl methacrylate copolymer, a methacrylic acid-ethyl acrylate copolymer, etc. Preferably, the methacrylic acid-ethyl acrylate copolymer is used. For example, when Eudragit L100-55 is selected, the prepared tablet may achieve an ideal release effect.

The study of the present disclosure also finds that certain parameters in the preparation process affect the yield of the tablet of the present disclosure and whether the tablet reaches a targeted release mode, thereby affecting the practicability/promotable value of the technology of the present disclosure, such as the pre-pressing pressure and the main pressure of the tablet pressing in step (3) of the preparation method: when the pre-pressing pressure is more than 1.5 kN, such as 2 kN, most tablet core enteric coatings are damaged, the tablet core of the obtained tablet is released in an acid, and when the pre-pressing pressure is 0.02-1.5 kN, wrapping by the tablet core enteric coatings is complete and the tablet core is not released in an acid for 4 h, contributing to obtain an ideal release mode; when the main pressure lower than 15 kN, such as 10 kN, is used for tableting, the pressed tablet is relatively soft and easy to break, and the friability is not qualified; and when the main pressure is greater than 35 kN, the pressed tablet has a top crack phenomenon and an enteric coating film of the tablet core is cracked, such that an active drug in the tablet core is released in the acid. A test shows that when the main pressure is within a range of 15-35 kN, the friability is qualified, wrapping by the tablet core enteric coatings is complete, and the tablet core is not released in the acid for 4 h.

When the medicinally active substance is a sleep-regulating drug, the test of the present disclosure shows that when a content ratio of a pharmaceutically active ingredient in the drug-containing immediate-release shell to the drug-containing delayed-release tablet core is (1-3): 1, an ideal blood concentration state may be realized. If the ratio of the drug in the drug-containing delayed-release tablet core is too high, a patient is not easy to wake up in a later period. If the ratio of the drug in the drug-containing delayed-release tablet core is too low and the ratio in the immediate-release shell is too high, an increase of the blood concentration is too high and too fast, the blood concentration in the later stage is low, and a blood concentration fluctuation is too large.

### Brief Description of Drawings

FIG. 1 is a cross-sectional structural schematic diagram of the tablet of the present disclosure, wherein 1-drug-containing delayed-release tablet core, 2-drug-free stomach-soluble coating, 3-drug-free enteric coating, 4-drug-containing immediate-release shell, and 5-shell stomach-soluble coating;
FIG. 2 shows a result of a dissolution test of the tablet obtained in example 1;
FIG. 3 shows a result of a dissolution test of the tablet obtained in example 2;
FIG. 4 shows a result of a dissolution test of the tablet obtained in example 3;
FIG. 5 shows a result of a dissolution test of the tablet obtained in example 4;
FIG. 6 shows an effect of a pre-pressing pressure on a tablet drug release model in a preparation process of the tablet of the present disclosure;
FIG. 7 shows an effect of a main-pressing pressure on a tablet drug release model in a preparation process of the tablet of the present disclosure;
FIG. 8 shows an effect of an enteric coating material on a drug release model of the tablet of the present disclosure;
FIG. 9 shows an effect of an enteric coating material weight gain on a drug release model of the tablet of the present disclosure; and
FIG. 10 shows an *in-vivo* release data comparison of the tablet and the reference preparation of the present disclosure.

### Detailed Description of Embodiments

In order to make the objective, technical solutions and advantages of the present application clearer, the technical solutions in the examples of the present application will be described in more detail below with reference to the drawings in the examples of the present application.

As shown in FIG. 1:
1. A tablet allowing release by stages of a drug, wherein the tablet contains a drug-containing delayed-release tablet core (1), a drug-free enteric coating (3), a drug-containing immediate-release shell (4), and a shell stomach-soluble coating (5) sequentially from inside to outside.
2. Preferably, in the tablet, a drug-free stomach-soluble coating (2) is arranged between the drug-containing delayed-release tablet core (1) and the drug-free enteric coating (3).
3. Preferably, in the tablet, a difference between the diameter of the tablet and the diameter of the drug-containing delayed-release tablet core (1) is ≥ 2 mm.
4. Preferably, in the tablet, a difference between the diameter of the tablet and the diameter of the drug-containing delayed-release tablet core (1) is between 2-8 mm or 2-6 mm or 3-7 mm or 4-8 mm.
5. The tablet according to claim 1, a difference between the thickness of the tablet and the thickness of the drug-containing delayed-release tablet core (1) is ≥ 1mm.
6. Preferably, in the tablet, a difference between the thickness of the tablet and the thickness of the drug-containing delayed-release tablet core (1) is between 1-8 mm or 1.5-6 mm or 2-6 mm.
7. Preferably, in the tablet, the diameter of the tablet is 5-13 mm or 9.9-10.25 mm; and the diameter of the drug-containing delayed-release tablet core (1) is 3-9 mm or 4.9-5.25 mm.
8. Preferably, in the tablet,
   the thickness of a narrower side from an upper surface of the drug-containing delayed-release tablet core (1) to an upper surface of the tablet is ≥ 0.5 mm; and
   the thickness of a narrower side between an outer edge of the drug-containing delayed-release tablet core (1) and an outer edge of the tablet is ≥ 0.5 mm.
9. Preferably, in the tablet, in a longitudinal section direction of the tablet, the drug-containing delayed-release tablet core (1) is located in a geometrically central region of the tablet; and
   in a cross section direction of the tablet, the drug-containing delayed-release tablet core (1) is located in a geometrically central region of the tablet.
10. Preferably, in the tablet, the drug refers to a sleep-regulating drug, such as ramelteon.

In an example of a typical tablet of the present disclosure, the tablet core is pressed using a punching die having a diameter of 5 mm, such that the theoretical diameter of the drug-containing tablet core in an uncoated state should be 5 mm. The whole tablet is pressed using a punching die having a diameter of 10 mm, such that the theoretical diameter of the whole tablet in an uncoated state is 10 mm. The tablet core is theoretically in the middle of the whole tablet, such that theoretically, but actually, a slight error exists. A vertical distance from a periphery of the tablet core to a periphery of the tablet does not affect a final release curve within a range of 0.5-4.5 mm.

### Example 1 Preparation of tablet (the delayed-release tablet core is immediate-release)

| Preparation composition | | **Example 1** | |
|---|---|---|---|
| | | Prescription ratio% | Prescription dosage per unit mg |
| Delayed-release tablet core | Drug: Ramelteon | 6.67 | 4 |
| | Filler Lactose | 68.81 | 41.29 |
| | Filler MCC | 15 | 9 |
| | Binder PVP/VA | 2.5 | 1.5 |
| | Disintegrant CCMC-Na | 6 | 3.6 |
| | Antioxidant BHT | 0.02 | 0.012 |
| | Lubricant/flow aid (magnesium stearate) | 1 | 0.6 |
| | Total | 100 | 60 |
| | | Weight gain ratio% | Prescription dosage per unit mg |
| Tablet core coating | Opadry^{®} 85F38197-CN | 2 | 1.2 |
| | Eudragit L 30D-55 | 6 | 3.67 |
| | | Prescription ratio% | Prescription dosage per unit mg |
| Immediate-release outer shell | Ramelteon | 1.14 | 4 |
| | Lactose | 74.34 | 260.18 |
| | MCC | 15 | 52.5 |
| | PVP/VA | 2.5 | 8.75 |
| | CCMC-Na | 6 | 21 |
| | BHT | 0.02 | 0.07 |
| | Magnesium stearate | 1 | 3.5 |
| | Total | 100 | 350 |
| | | Weight gain ratio% | Prescription dosage per unit mg |
| Coating | Opadry^{®} 85F38197-CN | 3 | 12.45 |
| Total | | / | 427.32 |

A preparation method was as follows:
(1) an immediate-release composition was prepared, sieved internal auxiliary materials (lactose, MCC, and PVPN A) were weighed according to the prescription dosage, ramelteon and BHT were added into a certain amount of ethanol to prepare a solution, the solution was subjected to a wet granulation, the granule was dried, sieved CCMC-Na and magnesium stearate were added, and the materials were uniformly mixed;
(2) a delayed-release composition was prepared, ramelteon was weighed according to the prescription dosage of a main drug, sieved auxiliary materials were weighed according to an auxiliary material formula, and the materials were fully and uniformly mixed according to an equal amount adding method;
(3) a tablet core was pressed by using a delayed-release preparation composition and subjected to stomach-soluble coating and enteric coating; and
(4) an immediate-release preparation composition was used as a shell and the delayed-release tablet core was used as a tablet core to press a core-wrapped tablet, and the immediate-release composition was used as a first layer to be filled into a middle die; the delayed-release tablet core was placed on the first layer and pre-pressed (0.02 kN); and the immediate-release composition was filled again as a third layer and pressed into a tablet with a main pressure of 20 kN.

Dissolution conditions were as follows: Chinese Pharmacopoeia 2015 Edition Part VI General Rules 0931 Method II, a paddle method of 50 rpm, 37°C, and 500 mL of a medium with a pH of 1.0/a medium with a pH of 7.2, and determination at 210 nm by using an ultraviolet (UV) spectrophotometry.

A test result of a dissolution rate was shown in FIG. 2 that the immediate-release shell was completely released within 15 minutes; and no drug was released during a period from 15 minutes later to 120 minutes earlier, and the delayed-release tablet core was completely released within 30 minutes after 120 minutes.

### Example 2 Preparation of tablet (delayed-release tablet core is slow-release)

| Preparation composition | | **Example 2** | |
|---|---|---|---|
| | | Prescription ratio% | Prescription dosage per unit mg |
| Delayed-release tablet core | Ramelteon | 10.00 | 6 |
| | Filler Calcium hydrogen phosphate | 51.45 | 30.87 |
| | Filler MCC | 15 | 9 |
| | Binder PVP | 2.5 | 1.5 |
| | Slow-release material HPMC | 20 | 12 |
| | Antioxidant BHA | 0.05 | 0.03 |
| | Lubricant/flow aid (magnesium stearate) | 1 | 0.6 |
| | Total | 100 | 60 |
| | | Weight gain ratio% | Prescription dosage per unit mg |
| Tablet core coating | Opadry^{®} 40L180000 | 2 | 1.2 |
| | Acryl^{®} 93084720 | 8 | 4.90 |
| | | Prescription ratio% | Prescription dosage per unit mg |
| Immediate-release outer shell | Ramelteon | 0.57 | 2 |
| | Lactose | 69.38 | 242.83 |
| | MCC | 20 | 70 |
| | PVP | 3 | 10.5 |
| | Crospovidone | 6 | 21 |
| | BHA | 0.05 | 0.18 |
| | Magnesium stearate | 1 | 3.5 |
| | Total | 100 | 350 |
| | | Weight gain ratio% | Prescription dosage per unit mg |
| Coating | Opadry^{®} 40L180000 | 3 | 12.48 |
| Total | | / | 428.58 |

A preparation method was as follows:
(1) an immediate-release composition was prepared, ramelteon and sieved internal auxiliary materials (lactose, MCC, and BHA) were weighed according to the prescription dosage, PVP was added into a certain amount of water to prepare a binder, a wet granulation was performed, the granule was dried, sieved crospovidone and magnesium stearate were added, and the materials were uniformly mixed;
(2) a delayed-release composition was prepared, ramelteon and sieved internal auxiliary materials (calcium hydrogen phosphate, MCC, HPMC, and BHA) were weighed according to the prescription dosage, PVP was added into a certain amount of water to prepare a binder, a wet granulation was performed, the granule was dried, sieved magnesium stearate was added, and the materials were uniformly mixed;
(3) a tablet core was pressed by using a delayed-release preparation composition and subjected to stomach-soluble coating and enteric coating; and
(4) an immediate-release preparation composition was used as a shell and the delayed-release tablet core was used as a tablet core to press a core-wrapped tablet, and the immediate-release composition was used as a first layer to be filled into a middle die; the delayed-release tablet core was placed on the first layer and pre-pressed (0.04 kN); and the immediate-release composition was filled again as a third layer and pressed into a tablet with a main pressure of 25 kN.

Dissolution conditions were as follows: Chinese Pharmacopoeia 2015 Edition Part VI General Rules 0931 Method II, a paddle method of 50 rpm, 37°C, and 500 mL of a medium with a pH of 1.0/a medium with a pH of 6.8, and determination at 210 nm by using an ultraviolet (UV) spectrophotometry.

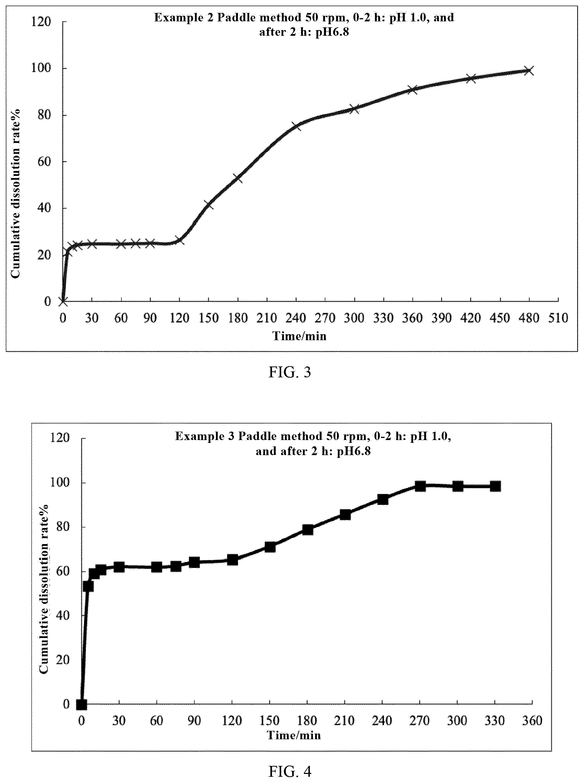

A test result of a dissolution rate was shown in FIG. 3.

The immediate-release shell was completely released within 10 minutes; no drug was released during a period from 15 minutes later to 120 minutes earlier, and the delayed-release tablet core started to release a pharmaceutical ingredient after 120 minutes continuously to the 10th hour. Under the formula, if the ratio of the drug contained in the delayed-release tablet core was too high, a person taking the drug is not easy to wake up in a later period.

### Example 3 Preparation of tablet (delayed-release tablet core is slow-release)

| Preparation composition | | | **Example 3** | |
|---|---|---|---|---|
| | | | Prescription ratio% | Prescription dosage per unit mg |
| Delayed-release tablet core | | Ramelteon | 5 | 3 |
| | | Filler Mannitol | 44.90 | 26.94 |
| | | Filler MCC | 40 | 24 |
| | | Binder PVP/VA | 4 | 2.4 |
| | Slow-release material Sodium alginate | | 5 | 3 |
| | Antioxidant VC | | 0.1 | 0.06 |
| | Lubricant/flow aid Colloidal silicon dioxide | | 0.5 | 0.3 |
| | Lubricant/flow aid Sodium stearyl fumarate | | 0.5 | 0.3 |
| | Total | | 100 | 60 |

| | | | Weight gain ratio% | Prescription dosage per unit mg |
|---|---|---|---|---|
| Tablet core coating | | Opadry^{®} 03K170007-CN | 2 | 1.2 |
| | | Eudragit L100 | 7 | 4.28 |

| | | | Prescription ratio% | Prescription dosage per unit mg |
|---|---|---|---|---|
| Immediate-release outer shell | | Ramelteon | 1.43 | 5 |
| | | MCC | 71.47 | 250.15 |
| | | Mannitol | 15 | 52.5 |
| | | PVP/VA | 3 | 10.5 |
| | | Sodium carboxymethyl starch | 8 | 28 |
| | | VC | 0.1 | 0.35 |
| | | Colloidal silicon dioxide | 0.5 | 1.75 |
| | | Sodium stearyl fumarate | 0.5 | 1.75 |
| | | Total | 100 | 350 |
| | | | Weight gain ratio% | Prescription dosage per unit mg |
| Coating | | Opadry-stomach-soluble 03K170007-CN | 3 | 12.48 |
| Total | | | / | 428.58 |

A preparation method was as follows:
(1) an immediate-release composition was prepared, ramelteon was weighed according to the prescription dosage of a main drug, sieved auxiliary materials were weighed according to an auxiliary material formula, and the materials were fully and uniformly mixed according to an equal amount adding method;
(2) a delayed-release composition was prepared, ramelteon was weighed according to the prescription dosage, sieved auxiliary materials were weighed according to an auxiliary material formula, and the materials were fully and uniformly mixed according to an equal amount adding method;
(3) a tablet core was pressed by using a delayed-release preparation composition and subjected to stomach-soluble coating and enteric coating; and
(4) an immediate-release preparation composition was used as a shell and the delayed-release tablet core was used as a tablet core to press a core-wrapped tablet, and the immediate-release composition was used as a first layer to be filled into a middle die; the delayed-release tablet core was placed on the first layer and pre-pressed (0.02 kN); and the immediate-release composition was filled again as a third layer and pressed into a tablet with a main pressure of 17 kN.

Dissolution conditions were as follows: Chinese Pharmacopoeia 2015 Edition Part VI General Rules 0931 Method II, a paddle method of 50 rpm, 37°C, and 500 mL of a medium with a pH of 1.0/a medium with a pH of 6.8, and determination at 210 nm by using an ultraviolet (UV) spectrophotometry.

A test result of a dissolution rate was shown in FIG. 4.

The immediate-release shell was completely released within 10 minutes; no drug was released during a period from 15 minutes later to 120 minutes earlier; and the delayed-release tablet core started to release a pharmaceutical ingredient after 120 minutes continuously to the 5th hour to reach a release of 100% to reach an ideal release mode.

### Example 4 Preparation of tablet (delayed-release tablet core is slow-release)

| Preparation composition | | | **Example 4** | |
|---|---|---|---|---|
| | | | Prescription ratio% | Prescription dosage per unit mg |
| Delayed-release tablet core | | Ramelteon | 5.83 | 3.5 |
| | | Filler Mannitol | 53.67 | 32.20 |
| | | Filler MCC | 25 | 15 |
| | | Binder HPC | 6 | 3.6 |
| | Slow-release material HPMC | | 8 | 4.8 |
| | Antioxidant VC | | 0.5 | 0.3 |
| | Lubricant/flow aid Sodium stearyl fumarate | | 1 | 0.6 |
| | | Total | 100 | 60 |
| | | | Weight gain ratio% | Prescription dosage per unit mg |
| Tablet core coating | Opadry^{®} 20A99172-CN | | 2 | 1.2 |
| | | Acryl^{®} 93018509 | 5 | 3.06 |
| | | | Prescription ratio% | Prescription dosage per unit mg |
| Immediate-release outer shell | | Ramelteon | 1.57 | 5.5 |
| | | MCC | 53.93 | 188.75 |
| | | Mannitol | 30 | 105 |
| | | PVP | 5 | 17.5 |
| | Sodium carboxymethyl starch | | 8 | 28 |
| | | VC | 0.5 | 1.75 |
| | | Sodium stearyl fumarate | 1 | 3.5 |
| | | Total | 100 | 350 |
| | | | Weight gain ratio% | Prescription dosage per unit mg |
| Coating | Opadry^{®} 20A99172-CN | | 3 | 12.43 |
| Total | | | / | 426.69 |

A preparation method was as follows:
(1) an immediate-release composition was prepared, sieved internal auxiliary materials (mannitol, MCC, PVP, VC, and sodium carboxymethyl starch) were weighed according to the prescription dosage, ramelteon was added into a certain amount of ethanol to prepare a solution, the solution was subjected to a wet granulation, the granule was dried, sieved sodium stearyl fumarate was added, and the materials were uniformly mixed;
(2) a delayed-release composition was prepared, ramelteon was weighed according to the prescription dosage of a main drug, sieved auxiliary materials were weighed according to an auxiliary material formula, and the materials were fully and uniformly mixed according to an equal amount adding method;
(3) a tablet core was pressed by using a delayed-release preparation composition and subjected to stomach-soluble coating and enteric coating; and
(4) an immediate-release preparation composition was used as a shell and the delayed-release tablet core was used as a tablet core to press a core-wrapped tablet, and the immediate-release composition was used as a first layer to be filled into a middle die; the delayed-release tablet core was placed on the first layer and pre-pressed with a pre-pressing pressure (0.03 kN); and the immediate-release composition was filled again as a third layer and pressed into a tablet with a main pressure of 20 kN.

Dissolution conditions were as follows: Chinese Pharmacopoeia 2015 Edition Part VI General Rules 0931 Method II, a paddle method of 50 rpm, 37°C, and 500 mL of a medium with a pH of 1.0/a medium with a pH of 7.2, and determination at 210 nm by using an ultraviolet (UV) spectrophotometry.

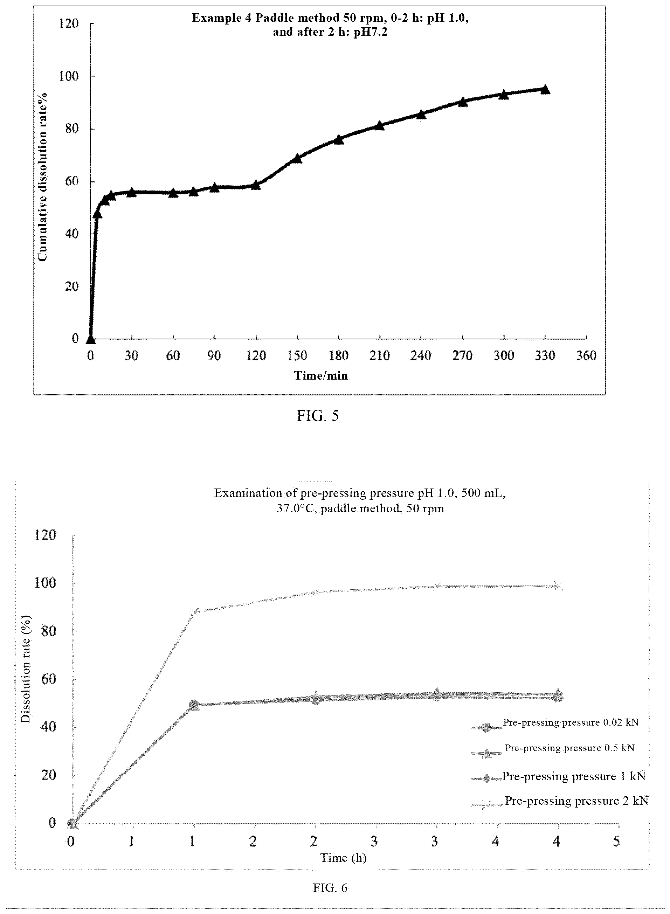

A test result of a dissolution rate was shown in FIG. 5. The immediate-release shell was completely released within 10 minutes; no drug was released during a period from 15 minutes later to 120 minutes earlier; and the delayed-release tablet core started to release a pharmaceutical ingredient after 120 minutes continuously to the 5.5th hour to reach a release of 100% to realize a relatively ideal release mode.

### Test1. Test Pre-pressing Pressure

During the research and development process, it was found that during the core-wrapped tablet pressing process, the pre-pressing pressure may affect an interface interaction and an adhesive force between the tablet core and the immediate-release shell, and may also affect the integrity of the enteric coating film of the tablet core, thereby affecting the dissolution of a sample.

An effect of different pre-pressing pressures on a dissolution effect was investigated using the prescription and the preparation method of example 4.

Different pre-pressing pressures (0.02 kN, 0.5 kN, 1 kN, and 2 kN) were respectively used to perform prepressing; and the immediate-release composition was filled as a third layer again and pressed into a tablet with a main pressure of 20 kN.

Dissolution conditions were as follows: Chinese Pharmacopoeia 2015 Edition Part VI General Rules 0931 Method II, a paddle method of 50 rpm, 37°C, and 500 mL of a medium with a pH of 1.0/a medium with a pH of 6.8, and determination at 210 nm by using an ultraviolet (UV) spectrophotometry.

As shown in FIG. 6, when the pre-pressing pressure was 0.02, 0.5, and 1 kN, the wrapping by the tablet core enteric coating was complete, the tablet core was not released in an acid for 4 h, and when the pre-pressing pressure was greater than 1.5 kN such as 2 kN, the wrapping by the tablet core enteric coating was incomplete and damaged, and the tablet core was released in the acid.

### Test 2. Test Main Pressure

In the research and development process, it was found that when the main pressure was within a range of 15-35 kN, the friability was qualified, wrapping by the tablet core enteric coatings was complete, and the tablet core was not released in an acid for 4 h. The prescription and the tableting process of example 1 were used except that the main pressure was set to be 15, 20, 25, and 35 kN respectively. An effect of the main pressure on a dissolution effect was studied.

Dissolution conditions were as follows: Chinese Pharmacopoeia 2015 Edition Part VI General Rules 0931 Method II, a paddle method of 50 rpm, 37°C, and 500 mL of a medium with a pH of 1.0/a medium with a pH of 6.8, and determination at 210 nm by using an ultraviolet (UV) spectrophotometry.

A test result was shown in FIG. 7. When the tablet was pressed with a main pressure of 10 kN, the tablet was cracked and the friability was not qualified. When the main pressure was greater than 35 kN (such as 35 kN), the enteric coating film of the tablet core was cracked, such that an active drug in the tablet core was released in the acid.

### Test 3. Comparison of enteric coating material

An effect of different enteric coating materials was compared.

The prescription process of example 4 was used to examine different enteric coating materials respectively.

Dissolution method: a paddle method of 50 rpm, 37°C, and 500 mL of a medium with a pH of 5.0, and determination at 210 nm by using an ultraviolet (UV) spectrophotometry.

A test result was shown in FIG. 8. When the coating material was Opadry enteric 94O680000 and 910610002, the enteric coating film may be less tough or may not remain soluble at a pH of 5.0. When Eudragit L100-55 was used as the coating material, a main film-forming material was a methacrylic acid and ethyl acrylate copolymer and an ideal release effect may be achieved.

### Test 4. Comparison of enteric coating weight gain and thickness

The prescription of example 1 was used to study and compare a tableting effect of coating weight gains of 3%, 4%, 5%, 6%, 7%, 8%, 10%, and 12%.

Compared with different enteric coating weight gain thicknesses, when a weight gain range was 4-12%, the dissolution was similar. A second-stage release reached a platform within 0.5-1 h, so as to reach an expected dissolution effect.

Dissolution conditions were as follows: Chinese Pharmacopoeia 2015 Edition Part VI General Rules 0931 Method II, a paddle method of 50 rpm, 37°C, and 500 mL of a medium with a pH of 1.0/a medium with a pH of 6.8, and determination at 210 nm by using an ultraviolet (UV) spectrophotometry.

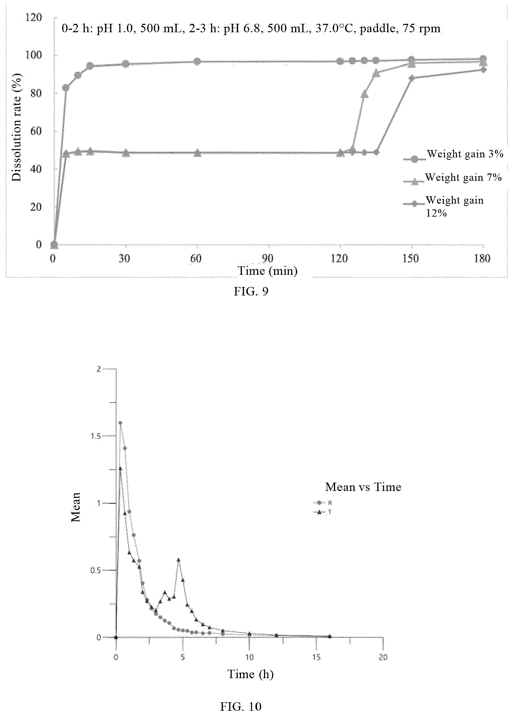

A result was shown in FIG. 9. If the coating film was too thin, the tablet core cannot be protected from releasing in an acid, and an enteric effect cannot be achieved. If the coating film was too thick, a tableting cost was increased.

### Test 5. In-vivo data

A human body test comparison of a sample (T) prepared in the **Example 1** with a reference preparation (R) was performed, wherein the reference preparation (R) was a ramelteon tablet (Rozerem^{®}) with a specification of 8 mg, a batch number of HH2012, a manufacturer of Japan Takeda Pharmaceuticals, and preservation conditions of shading, sealing, and storing at below 25°C.

A single-center, random, open, two-cycle, double-crossover, and single-dose test design method was used. The dose was 1 tablet/person, a washing period was 2 days, and 8 subjects completed the test.

A test result was shown in FIG. 10, wherein the sample (T) of the present disclosure had a first release Cₘₐₓ smaller than the reference preparation (R), which may reduce the incidence of adverse reactions. Besides, the sample (T) of the present disclosure still had another release at about 5 h compared to the reference preparation (R), which may effectively prevent early awakening.

## Claims

**1.** A tablet allowing sleep-regulating active ingredients release by stages, wherein each tablet structurally from inside to outside sequentially contains a drug-containing delayed-release tablet core, a drug-free enteric coating, a drug-containing immediate-release shell, and a shell coating.

**2.** The tablet according to claim 1, wherein a film-forming material of the drug-free enteric coating is selected from acrylic resin I, II, and III, cellulose acetate phthalate (CAP), hydroxypropyl methylcellulose phthalate (HPMCP), polyvinyl acetate phthalate (PVAP), cellulose acetate trimellitate (CAT), acrylic resin EuS100 and EuL100, a methacrylic acid-methyl methacrylate copolymer, and a methacrylic acid-ethyl acrylate copolymer, and preferably, the used film-forming material is the methacrylic acid-ethyl acrylate copolymer.

**3.** The tablet according to claim 2, wherein the drug-free enteric coating provides a weight gain by > 4% on the basis of the drug-containing delayed-release tablet core, and the drug-free enteric coating is soluble at a pH of 5 or more, such that 0.5-4 hours after the drug-containing immediate-release shell finishes release, the drug-containing delayed-release tablet core can start to release and release completely within 0.1-6 hours.

**4.** The tablet according to claim 3, wherein the drug-free enteric coating provides a weight gain by 4-12%, 5%, 6%, 7% or 8% on the basis of the drug-containing delayed-release tablet core, and the drug-free enteric coating is soluble at a pH of 7 or above, such that 0.5-4 hours after the drug-containing immediate-release shell finishes release, the drug-containing delayed-release tablet core can start release and release completely within 0.5-4 hours.

**5.** The tablet according to claim 1, wherein , a drug-free stomach-soluble coating is arranged between the drug-containing delayed-release tablet core and the drug-free enteric coating, and the the drug-free stomach-soluble coating provides weight gain by 2-5% on the basis of the drug-containing delayed-release tablet core, preferably provides weight gain by 3% or 4%.

**6.** The tablet according to claim 1, wherein a content ratio of active ingredients in the drug-containing immediate-release shell to that in the drug-containing delayed-release tablet core is 1: 1 ~ 3:1.

**7.** The tablet according to claim 1, wherein the drug-containing delayed-release tablet core is an immediate-release preparation, in which auxiliary materials and corresponding weight percentage are respectively as follows:
60-90% of a filler;
2-10%, preferably 4-8% of a disintegrant;
2-6%, preferably 2.5-5% of a binder;
0.01-1%, preferably 0.05-1% of an antioxidant; and
0.5-3%, preferably 0.5-1.5% of a lubricant/a flow aid,
wherein the filler and the antioxidant are internal auxiliary materials, the lubricant/the flow aid is an external auxiliary material, and the disintegrant can be as internal auxiliary material or external auxiliary material.

**8.** The tablet according to claim 1, wherein the drug-containing delayed-release tablet core is a slow-release preparation, in which auxiliary materials and corresponding weight percentage are respectively as follows:
60-90% of a filler;
2-6%, preferably 2.5-5% of a binder;
4-25%, preferably 5-20% of a slow-release material;
0.01-1%, preferably 0.05-1% of an antioxidant; and
0.5-3%, preferably 0.5-1.5% of a lubricant ora flow aid,
wherein the filler, the binder, and the antioxidant are internal auxiliary materials, the lubricant orthe flow aid is external auxiliary material.

**9.** The tablet according to any one of claims 1-8, wherein a weight ratio of the drug-containing immediate-release shell to the drug-containing delayed-release tablet core is 4 or more, preferably in the range of 4~12, or is 350:60.

**10.** The tablet according to claim 7 or 8,
wherein the filler is one or more selected from microcrystalline cellulose, lactose, pregelatinized starch, starch, calcium hydrogen phosphate, mannitol, and dextrin;
the binder is selected from one or more ofpolyvidone, copovidone, hydroxypropyl cellulose, hydroxypropyl methylcellulose;
the disintegrant is selected from one or more of starch, pregelatinized starch, low-substituted hydroxypropyl cellulose, sodium carboxymethyl starch, crospovidone, and croscarmellose sodium;
the slow-release material is selected from one or more of hydroxypropyl cellulose, hydroxypropyl methylcellulose, alginate, ethyl cellulose, vinyl acetate, xanthan gum, guar gum, crospovidone, and glyceryl behenate;
the antioxidant is one or more selected from vitamin E, p-hydroxyanisole, butylated hydroxyanisole, dibutyl cresol, sodium bisulfite, sodium metabisulfite, and vitamin C; and
the lubricant/the flow aid is selected from one or more of magnesium stearate, sodium stearyl fumarate, colloidal silicon dioxide, sodium lauryl sulfate, and talcum powder.

**11.** The tablet according to any one of claims 1-10, prepared by using the following steps:
(1) preparing a material of the drug-containing immediate-release shell: preparing a total mixed particle of the drug-containing immediate-release shell according to a formula;
(2) preparing the drug-containing delayed-release tablet core: preparing a total mixed particle of the drug-containing delayed-release tablet core, and preparing a tablet core by using a pressing method; and sequentially performing stomach-soluble coating and enteric coating on the tablet core to obtain the drug-containing delayed-release tablet core; and
(3) filling a part of the material of the drug-containing immediate-release shell into a middle die as a first layer, and placing the drug-containing delayed-release tablet core on around middle area of the first layer to serve as second layer, then performing pre-pressing; and filling the rest material of the drug-containing immediate-release shell to cover the drug-containing delayed-release tablet core, and performing main-pressing to obtain the tablet.

**12.** The tablet according to claim 11, wherein the pre-pressing is performed at a pressure of 0.02-1.5 kN, and the main-pressing is performed at a pressure of 15-35 kN.

**12.** The tablet according to any one of claims 1-12, wherein the sleep-regulating active ingredients is ramelteon.

**13.** A method for preparing the tablet according to claims 1-12, comprising the following steps:
(1) preparing a material of the drug-containing immediate-release shell: preparing a total mixed particle of the drug-containing immediate-release shell according to a formula;
(2) preparing the drug-containing delayed-release tablet core: preparing a total mixed particle of the drug-containing delayed-release tablet core, and preparing a tablet core by using a pressing method; and sequentially performing stomach-soluble coating and enteric coating on the tablet core to obtain the drug-containing delayed-release tablet core; and
(3) filling a part of the material of the drug-containing immediate-release shell into a middle die as a first layer, and placing the drug-containing delayed-release tablet core on the first layer to serve as second layer, and then performing pre-pressing; and filling the rest material of the drug-containing immediate-release shell as a third layer, and performing main-pressing to obtain the tablet., ; and
preferably, in the preparation method, the pre-pressing is performed at a pressure of 0.02-1.5 kN, and the main-pressing is performed at a pressure of 15-35 kN.

**14.** The preparation method according to claim 13, wherein the total mixed particle of the drug-containing delayed-release tablet core and the total mixed particle of the drug-containing immediate-release shell are prepared by using any one of the following methods:
method I: respectively weighing the pharmaceutically effective substance and the various sieved internal auxiliary materials according to the prescription dosage, adding the pharmaceutically effective substance and the antioxidant into a certain amount of a solvent to prepare a solution or a suspension, spraying the solution or the suspension into the rest internal auxiliary materials to complete a wet granulation, adding the external auxiliary materials after drying, and uniformly mixing the materials to obtain a corresponding total mixed particle;
method II: respectively weighing the pharmaceutically effective substance and the various sieved internal auxiliary materials according to the prescription dosage, adding the pharmaceutically effective substance into a certain amount of a solvent to prepare a solution or a suspension, spraying the solution or the suspension into the internal auxiliary materials to complete a wet granulation, adding the external auxiliary materials after drying, and uniformly mixing the materials to obtain a corresponding total mixed particle;
method III: respectively weighing the pharmaceutically effective substance and the various sieved internal auxiliary materials according to the prescription dosage, adding the antioxidant and or the binder into a certain amount of a solvent to prepare a solution or a suspension, spraying the solution or the suspension into a mixed material of the pharmaceutically effective substance and the rest internal auxiliary materials to complete a wet granulation, adding the external auxiliary materials after drying, and uniformly mixing the materials to obtain a corresponding total mixed particle; and
method IV: respectively weighing the pharmaceutically effective substance and the various sieved internal auxiliary materials according to the prescription dosage, and uniformly mixing the pharmaceutically effective substance and the auxiliary materials according to an equal amount adding method to obtain a corresponding total mixed particle.
